# EUROPEAN PATENT APPLICATION

(11) **EP 3 945 320 A1**
(43) Date of publication of application: **02.02.2022**
(21) Application number: 20382685.4
(22) Date of filing: 29.07.2020
(51) Int. Cl.: G01N 33/574, G01N 33/543, C07K 7/04, C07K 7/08, C07K 17/00

(54) **IMPROVED EPITOPE FOR DETECTING AND/OR QUANTIFYING AUTOANTIBODIES AGAINST ALPHA-FETOPROTEIN**

(71) Applicant: Corporació Sanitària Parc Taulí, 08208 Sabadell (ES)
(72) Inventor: DELGADO DE LA POZA, JUAN FRANCISCO, 08208 Sabadell (ES); GALLEGO MORENO, FRANCESC XAVIER, 08208 Sabadell (ES)
(74) Representative: Hoffmann Eitle

(57) **Abstract**

A specific epitope has been identified in the present invention, which is herein used for detecting and/or quantifying autoantibodies against alpha-fetoprotein (AFP) in a biological sample, and consequently for the *in vitro* diagnosis of hepatocellular carcinoma (HCC).

## Description

### FIELD OF THE INVENTION

The present invention refers to the medical field. Particularly, a specific epitope has been identified in the present invention, which is herein used for detecting and/or quantifying autoantibodies against alpha-fetoprotein (AFP) in a biological sample, and consequently for the *in vitro* diagnosis of hepatocellular carcinoma (HCC).

### STATE OF THE ART

The HCC is the second cause of mortality related to cancer, and the sixth most common cause of cancer worldwide. Most patients diagnosed of HCC have liver cirrhosis as a baseline disease, thus mandatory HCC screening is performed with ultrasound every six months in all these patients. However, most patients, even those included in a screening program are diagnosed at late stages, when survival prognosis is worst. Accordingly, the survival rate at 5 years in patients with HCC is less than 16%. In order to improve the survival of these patients, it is very important to develop new tools to better diagnose HCC at early stages, when curative treatments are possible. Discovery of new biomarkers or the improvement of those already described to detect HCC at early stages will be essential to develop better screening tests. The *American Association for the Study of Liver Diseases* and the *European Association for the Study of the Liver* call for the need to establish screening tests for at-risk patients, mainly those suffering from cirrhosis. The screening protocol approved by both associations consists of abdominal ultrasound every 6 months. Other associations like British and Asian add AFP determination to ultrasound. Although, a recent meta-analysis sets the ultrasound sensitivity to detect HCC in 84%, the sensitivity drops to 47% when used for early detection. This meta-analysis also studies the comparison of sensitivity between ultrasound with or without serum AFP detection as screening test. Results show that ultrasound without serum AFP detection has a sensitivity of 78% to detect HCC, while the sensitivity increases to 97% when both ultrasound and serum AFP detection are concurrently used. However, early stage HCC detection shows a sensitivity of 45% when using ultrasound without serum AFP detection, and a sensitivity of 63%; when adding serum AFP detection to the ultrasound. Also, serum AFP detection alone shows a sensitivity of 46-59% and a specificity of 87-93% to early detect HCC. Appropriate screening leads to early diagnosis, which leads to better management options, a higher proportion of treatable lesions, and better outcomes, including survival. Screening at-risk population to develop HCC also has been demonstrated to be cost-effective if the expected HCC risk exceeds 1.5% per year in patients with hepatitis C and 0.2% per year in patients with hepatitis B.
The immune system plays an important role controlling cancer. At early tumorigenesis stages tumor cells release proteins, peptides to the cell surface that produce a humoral response against them. These proteins and peptides are known as tumor-associated antigens (TAA). TAAs could be key proteins in transformation of cancer and transition to malignancy, thus becoming important as a therapeutic target molecule. Importantly, antibodies against TAAs could be detected few months or years before cancer diagnosis, helping diagnosis at early stages. 80-90% of HCC have liver cirrhosis, thus antibodies against TAAs could be detected in cirrhotic patients at high risk to develop HCC.
Although, there are many TAAs associated to HCC, they are not used in clinical practice due to their low sensitivity, thus not suitable for screening HCC in at-risk population. In contrast, AFP could be a good TAA to detect serum autoantibodies due to; 1) its high specificity in the diagnosis of HCC, 2) its use in immunoassays, and 3) its similar sensitivity compared to other TAAs previously reported to detect serum autoantibodies.
Consequently, the present invention aims to identify, and immunogenicity-optimize the AFP immunodominant epitope as a TAA to detect serum AFP autoantibodies in patients with HCC and liver cirrhosis.

### DESCRIPTION OF THE INVENTION

### Brief description of the invention

As explained above, a specific epitope has been identified in the present invention, which is herein used for detecting and/or quantifying autoantibodies against alpha-fetoprotein (AFP) in a biological sample, and consequently for the *in vitro* diagnosis of hepatocellular carcinoma (HCC).
Such as it is shown in the Examples, after a screening process based on immunoassay techniques of different amino acid peptides included in protein AFP, immunogenicity-optimized epitopes have been identified in the present invention.
Of note, SEQ ID NO: 1 of peptide 9.7.1 is present in several amino acid sequences which show good results. For instance, the SEQ ID NO: 1 of peptide 9.7.1 is included in SEQ ID NO: 2, SEQ ID NO: 3, SEQ ID NO: 12 and SEQ ID NO: 26 to 38. So, this sequence is herein presented as a key sequence for conferring immunogenicity to the epitope.
Moreover, a validation process was carried out as shown in the Examples and peptides 9.7.8 of SEQ ID NO: 2 and, particularly, peptide 9.7 of SEQ ID NO: 3, were confirmed as good epitopes for AFP determination and/or quantification.
Kindly note that SEQ ID NO: 1 is included in SEQ ID NO: 2 which is in turn included in SEQ ID NO: 3.
So, the first embodiment of the present invention refers to an epitope, or to an antigen comprising the epitope, wherein the epitope consisting of SEQ ID NO: 1 or a sequence having an identity of at least 95% with the SEQ ID NO: 1. In a preferred embodiment, the epitope consists of SEQ ID NO: 2 or a sequence having an identity of at least 95% with the SEQ ID NO: 2. In a still preferred embodiment, the epitope consists of SEQ ID NO: 3 or a sequence having an identity of at least 95% with the SEQ ID NO: 3.
The second embodiment of the present invention refers to an *in vitro* method for detecting and/or quantifying autoantibodies against alpha-fetoprotein (AFP) in a biological sample, wherein the autoantibodies bind to an epitope which comprises or consists of SEQ ID NO: 1, SEQ ID NO: 2 or SEQ ID NO: 3 and it is immobilized on a solid support. In a preferred embodiment, the autoantibodies bind to an antigen comprising an epitope which in turn comprises or consists of SEQ ID NO: 1, SEQ ID NO: 2 or SEQ ID NO: 3 and it is immobilized on a solid support. In a preferred embodiment, the detection and/or quantification of the autoantibodies is carried out by an immunochemical technique, preferably by ELISA, and most preferably by indirect ELISA, in which the antigen is bound by the primary antibody to be detected, that binds directly to the antigen, which then is detected by a labeled secondary antibody. So, the indirect ELISA is a two-step ELISA which involves two binding process of primary antibody and labeled secondary antibody. The primary antibody to be detected is incubated with the antigen followed by the incubation with the secondary antibody. Initially, micro-well plates are incubated with antigens characterized by comprising the epitope of the invention, washed up and blocked with BSA. After that, biological samples with may comprise the antibodies to be detected are added and washed. Moreover, enzyme linked secondary antibody are added and washed. A substrate is then added, and enzymes on the antibody elicit a chromogenic or fluorescent signal.
So, in a preferred embodiment, the present invention refers to an *in vitro* method which comprises:
a) Immobilizing an epitope comprising or consisting of the SEQ ID NO: 1, SEQ ID NO: 2 or SEQ ID NO: 3, or an antigen comprising an epitope which in turn comprises or consists of SEQ ID NO: 1, SEQ ID NO: 2 or SEQ ID NO: 3 on a solid support,
b) Adding the biological sample to be analysed in the solid support,
c) Adding an antibody, conjugated with a detectable labelling agent, which reacts against the autoantibody which may be present in the biological sample,
d) Detecting and/or quantifying the complex obtained with a composition containing a chromogenic, fluorogenic and/or chemiluminescent indicator substrate.

In a preferred embodiment, the sample is obtained from a subject who may be suffering from hepatocellular carcinoma.
In a preferred embodiment, the sample is blood, serum or plasma.
The third embodiment of the present invention refers to an *in vitro* method for the diagnosis of hepatocellular carcinoma which comprises detecting and/or quantifying autoantibodies against AFP by following the above described method.
The fourth embodiment of the present invention refers to the *in vitro* use of an epitope comprising or consisting of the SEQ ID NO: 1, SEQ ID NO: 2 or SEQ ID NO: 3, or an antigen comprising an epitope which in turn comprises or consists of SEQ ID NO: 1, SEQ ID NO: 2 or SEQ ID NO: 3 for detecting and/or quantifying autoantibodies AFP. In a preferred embodiment said epitope or antigen is used for the diagnosis of hepatocellular carcinoma.
The fifth embodiment of the present invention refers to a kit for detecting and/or quantifying autoantibodies against AFP in a biological sample which comprises:
a) An epitope comprising or consisting of the SEQ ID NO: 1, SEQ ID NO: 2 or SEQ ID NO: 3 or an antigen comprising an epitope which in turn comprises or consists of SEQ ID NO: 1, SEQ ID NO: 2 or SEQ ID NO: 3.
b) At least a secondary antibody which reacts against the autoantibody which may be present in the biological sample.

In a preferred embodiment this kit is intended for the diagnosis of hepatocellular carcinoma.
For the purpose of the present invention the following terms are defined:
- The term "comprising" means including, but not limited to, whatever follows the word "comprising". Thus, use of the term "comprising" indicates that the listed elements are required or mandatory, but that other elements are optional and may or may not be present.
- By "consisting of" is meant including, and limited to, whatever follows the phrase "consisting of'. Thus, the phrase "consisting of" indicates that the listed elements are required or mandatory, and that no other elements may be present.

### Detailed description of the invention

The present invention is illustrated by the Examples set below which merely serve as a proof of concept without the intention of limiting its scope.

### Example 1. Study subjects.

We selected 100 consecutive patients from outpatient hepatology unit of Parc Taulí University Hospital (68 males, 32 females, within the ages of 43-93 years old and median age of 74 years old). 58 patients had confirmed HCC following the criteria of the American Association for the Study of Liver Diseases (AASLD), 42 patients had confirmed hepatic cirrhosis from any etiology according to the criteria of AASLD. 40 non-hepatic diseases serum donors from Banc de Sang i Teixits de Barcelona were selected as a control group. The HCC group of patients was followed-up during 36.2±29.8 months and cirrhosis group was followed-up during 32.5±33.3 months. The ethical committee's approval was obtained, and all samples collected at our Hospital were accompanied by patient's consent. All patients signed an informed consent.

### Example 2. First phase of the epitope mapping.

AFP is a 609 amino acid protein. It binds copper, nickel, and fatty acids as well as, and bilirubin less well than, serum albumin. Amino acids 1-18 correspond to the signal peptide and positions 19-609 correspond to the AFP chain.
To make the first epitope mapping, 48 amino acid biotinylated peptides were synthesized with overlapping of 8 amino acids.
The peptides that were synthesized are included in **Table 1:**

**Table 1**

| **Peptide** | **Amino acid sequence** | **SEQ ID** |
|---|---|---|
| Peptide 1 | RTLHRNEYGIASILDSYQCTA | SEQ ID NO: 4 |
| | EISLADLATIFFAQFVQEATY KEVSKM | |
| Peptide 2 | TYKEVSKMVKDALTAIEKPT GDEQSAGCLENQLPAFLEEL CREKEILE | SEQ ID NO: 5 |
| Peptide 3 | CREKEILEKYGHSDCCSQSE EGRHNCFLAHKKPTPASIPF FQVPEPVT | SEQ ID NO: 6 |
| Peptide 4 | FQVPEPVTSCEAYEEDRETF MNKFIYEIARRHPFLYAPTI LLWAARYD | SEQ ID NO: 7 |
| Peptide 5 | LLWAARYDKIIPSCCKAEN AVECFQTKAATVTKELRES SLLNQHACAV | SEQ ID NO: 8 |
| Peptide 6 | LNQHACAVMKNFGTRTFQ AITVTKLSQKFTKVNFTEIQ KLVLDVAHVH | SEQ ID NO: 9 |
| Peptide 7 | VLDVAHVHEHCCRGDVLD CLQDGEKIMSYICSQQDTL SNKITECCKLT | SEQ ID NO: 10 |
| Peptide 8 | ITECCKLTTLERGQCIIHAEN DEKPEGLSPNLNRFLGDRDF NQFSSGE | SEQ ID NO: 11 |
| **Peptide 9** | FNQFSSGEKNIFLASFVHEY SRRHPQLAVSVILRVAKGY QELLEKCFQ | **SEQ ID NO: 12** |
| Peptide 10 | ELLEKCFQTENPLECQDKG EEELQKYIQESQALAKRSC GLFQKLGEYY | SEQ ID NO: 13 |
| Peptide 11 | FQKLGEYYLQNAFLVAYTK KAPQLTSSELMAITRKMAA TAATCCQLSE | SEQ ID NO: 14 |
| Peptide 12 | ATCCQLSEDKLLACGEGAA | SEQ ID NO: 15 |
| | DIIIGHLCIRHETTPVNPGVG QCCTSSYA | |
| Peptide 13 | QCCTSSYANRRPCFSSLVVD ETYVPPAFSDDKFIFHKDLC QAQGVALQ | SEQ ID NO: 16 |
| Peptide 14 | QAQGVALQTMKQEFLINLV KQKPQITEEQLEAVIADFSG LLEKCCQGQ | SEQ ID NO: 17 |
| Peptide 15 | LEKCCQGQEQEVCFAEEGQ KLISKTRAALGVMW | SEQ ID NO: 18 |

To carry out epitope mapping, 9 serum samples from patients with HCC, 3 serum samples from patients with cirrhosis who developed HCC in its follow-up, and 4 serum samples from blood donors were used. The different peptides were sensitized in an ELISA plate to perform an immunoassay following the protocol below:
- Streptavidin-ELISA plates preparation:
   - Nunc ELISA plates maxisorp H4BX (Nunc).
   - Sensitized 100 µL of streptavidin (Merck) at 20 µg/mL in Carbonate-Bicarbonate 50 mM pH9.
   - Incubate overnight at 4°C.
   - Discard streptavidin.
   - Block ELISA plates with BSA 5% (Merck) in PBS (Inova Diagnostics) 2 hours at room temperature.
- ELISA Plate sensitization with peptides:
   - Prepare a dilution of biotinylated peptide at 10 µg/mL in Carbonate-Bicarbonate 50 mM pH9.
   - Add 100 µL of peptide per well.
   - Incubate 1 hour at room temperature.
   - Wash 3 times with Washing solution (Grifols).
- ELISA assay:
   - Serum samples were diluted 1/100 with PBS-Tween 20 0.05% (Diluent buffer, Grifols).
   - 100 µL of diluted serum samples were added per well and incubated for 1 hour with shaking at room temperature.
   - Perform 3 washes with PBS-Tween 20 0.05% (Washing solution, Grifols).
   - Anti-human IgG antibody conjugated with HRP (Thermofisher Scientific) were diluted at 1/5000 in diluent buffer (Grifols).
   - 100 µL of anti-human IgG were incubated for 1 hour at room temperature
   - Perform 3 washes with PBS-Tween 20 0.05% (Washing solution, Grifols)
   - 100 µL of substrate (Thermofisher) were incubated for 30 minutes at room temperature.
   - Addition of 100 µL of stop solution (sulfuric acid 25% or Grifols)

Spectrophotometer reading at 450 nm and reference filter at 620 nm.

### Example 3. First assay results.

The results are expressed in optic density obtained from the ELISA described above and are summarized in **Table 2** below, wherein the 15 peptides disclosed above were analyzed. The mean of optical densities obtained by the different peptides in the group of patients was analyzed. This mean was higher for peptide 9 (0,320), which indicates that a greater number of antibodies bind to this sequence compared to the rest of the analyzed sequences. Therefore peptide 9 (SEQ ID NO: 12) showed the best results.

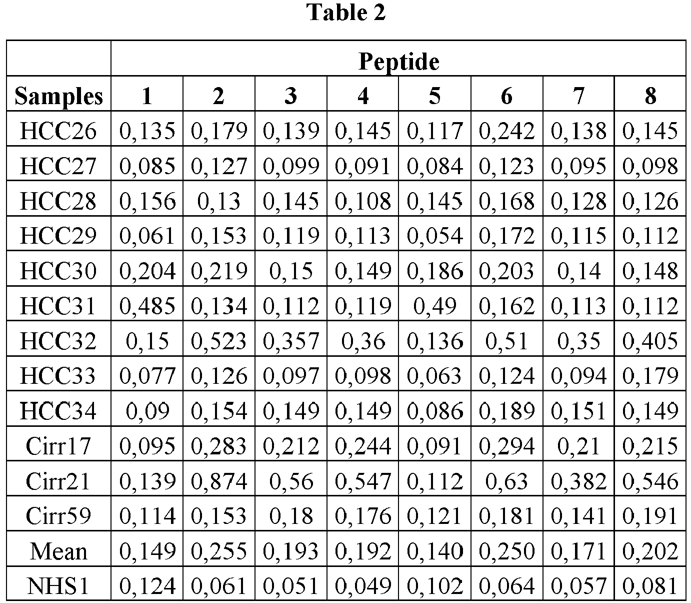

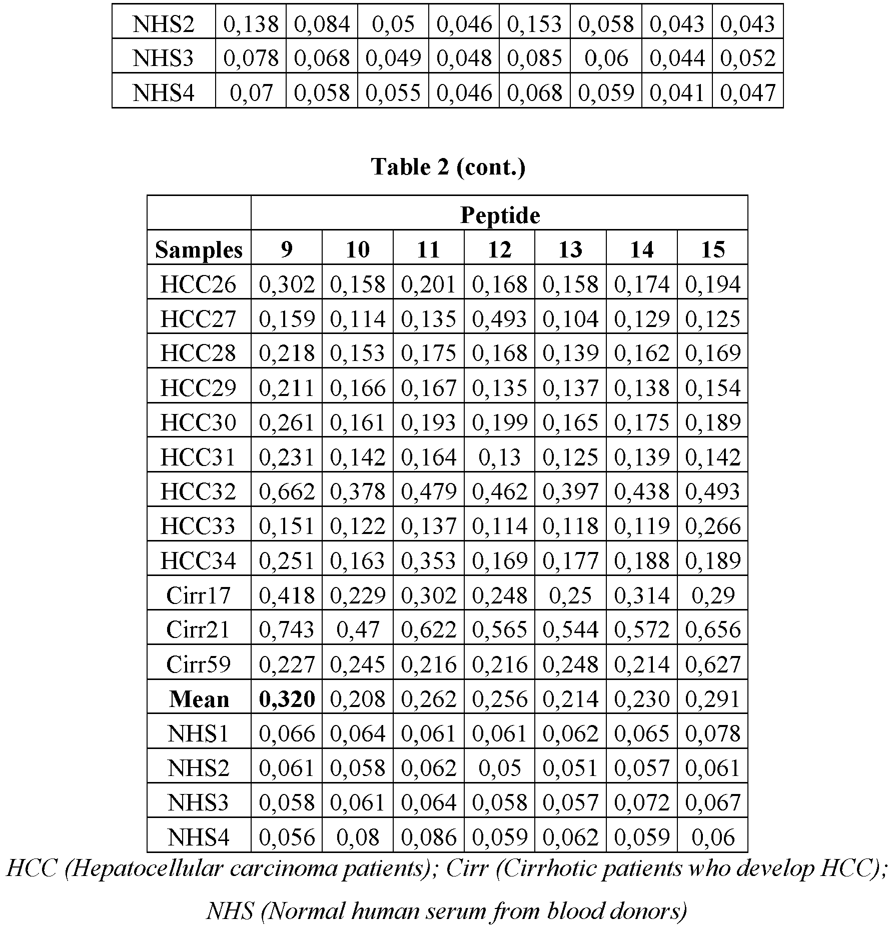

### Example 4. Second phase of epitope mapping.

Once the peptide 9 of SEQ ID NO: 12 was selected as the most interesting starting point, the second phase of epitope mapping was performed by decomposing the peptide 9, which comprises 48 amino acids, into shorter sequences of 20 amino acids with 16 overlapping amino acids. According with this strategy, the peptides which were synthesized are included in **Table 3.**

**Table 3**

| **Peptide** | **Amino acid sequence** | **SEQ ID** |
|---|---|---|
| Peptide 9.1 | FNQFSSGEKNIFLASFVHEY | SEQ ID NO: 19 |
| Peptide 9.2 | SSGEKNIFLASFVHEYSRRH | SEQ ID NO: 20 |
| Peptide 9.3 | KNIFLASFVHEYSRRHPQLA | SEQ ID NO: 21 |
| Peptide 9.4 | LASFVHEYSRRHPQLAVSVI | SEQ ID NO: 22 |
| Peptide 9.5 | VHEYSRRHPQLAVSVILRVA | SEQ ID NO: 23 |
| Peptide 9.6 | SRRHPQLAVSVILRVAKGYQ | SEQ ID NO: 24 |
| **Peptide 9.7** | **PQLAVSVILRVAKGYQELLE** | **SEQ ID NO: 3** |
| Peptide 9.8 | VSVILRVAKGYQELLEKCFQ | SEQ ID NO: 25 |

Then, the same immunoassay was performed with the same samples as described above in **Example 2.**

### Example 5. Second assay results.

The optic density obtained in the ELISA are summarized in **Table 4** below. The mean of optical densities obtained by the different peptides in the group of patients was analyzed. This mean was higher for peptide 9.7 (0,381), which indicates that a greater number of antibodies bind to this sequence compared to the rest of the analyzed sequences. Therefore, the peptide that shows the best results is peptide 9.7 of SEQ ID NO: 3.

**Table 4**

| | **Peptide** | | | | | | | |
|---|---|---|---|---|---|---|---|---|
| **Samples** | **9.1** | **9.2** | **9.3** | **9.4** | **9.5** | **9.6** | **9.7** | **9.8** |
| HCC26 | 0,102 | 0,087 | 0,079 | 0,077 | 0,083 | 0,077 | 0,423 | 0,097 |
| HCC27 | 0,096 | 0,09 | 0,076 | 0,077 | 0,087 | 0,074 | 0,348 | 0,082 |
| HCC28 | 0,309 | 0,167 | 0,16 | 0,164 | 0,163 | 0,16 | 0,411 | 0,176 |
| HCC29 | 0,065 | 0,048 | 0,047 | 0,047 | 0,05 | 0,049 | 0,35 | 0,057 |
| HCC30 | 0,072 | 0,045 | 0,045 | 0,044 | 0,049 | 0,047 | 0,261 | 0,059 |
| HCC31 | 0,228 | 0,206 | 0,196 | 0,21 | 0,205 | 0,205 | 0,416 | 0,234 |
| HCC32 | 0,437 | 0,422 | 0,443 | 0,415 | 0,421 | 0,411 | 0,589 | 0,429 |
| HCC33 | 0,041 | 0,04 | 0,041 | 0,072 | 0,04 | 0,04 | 0,044 | 0,041 |
| HCC34 | 0,084 | 0,069 | 0,071 | 0,067 | 0,072 | 0,068 | 0,368 | 0,086 |
| Cirr17 | 0,095 | 0,069 | 0,073 | 0,063 | 0,07 | 0,068 | 0,739 | 0,085 |
| Cirr21 | 0,116 | 0,089 | 0,085 | 0,083 | 0,089 | 0,089 | 0,812 | 0,107 |
| Cirr59 | 0,141 | 0,125 | 0,12 | 0,118 | 0,134 | 0,127 | 0,317 | 0,137 |
| **Mean** | 0,149 | 0,121 | 0,120 | 0,120 | 0,122 | 0,118 | **0,423** | 0,133 |
| NHS1 | 0,127 | 0,113 | 0,112 | 0,111 | 0,119 | 0,113 | 0,238 | 0,127 |
| NHS2 | 0,187 | 0,172 | 0,169 | 0,173 | 0,18 | 0,179 | 0,308 | 0,192 |
| NHS3 | 0,092 | 0,113 | 0,084 | 0,084 | 0,088 | 0,084 | 0,222 | 0,093 |
| NHS4 | 0,075 | 0,068 | 0,069 | 0,067 | 0,076 | 0,065 | 0,252 | 0,076 |

| | | | | | | | | |
|---|---|---|---|---|---|---|---|---|
| *HCC (Hepatocellular carcinoma patients); Cirr (Cirrhotic patients who develop HCC); NHS (Normal human serum from blood donors)* | | | | | | | | |

### Example 6. Third phase of epitope mapping.

Since the linear epitopes recognized by B lymphocytes comprise 5 to 20 amino acids, 15 more peptides were synthesized according to the results obtained in the second phase of epitope mapping to identify the epitope with the lowest number of amino acids **(see Table 5).**

**Table 5**

| **Peptide** | **Amino acid sequence** | **SEQ ID** |
|---|---|---|
| **Peptide 9.7.1** | **AVSVILRVAKGYQE** | **SEQ ID NO: 1** |
| Peptide 9.7.2 | LAVSVILRVAKGYQE | SEQ ID NO: 26 |
| Peptide 9.7.3 | AVSVILRVAKGYQEL | SEQ ID NO: 27 |
| Peptide 9.7.4 | QLAVSVILRVAKGYQE | SEQ ID NO: 28 |
| Peptide 9.7.5 | LAVSVILRVAKGYQEL | SEQ ID NO: 29 |
| Peptide 9.7.6 | AVSVILRVAKGYQELL | SEQ ID NO: 30 |
| Peptide 9.7.7 | AVSVILRVAKGYQELLE | SEQ ID NO: 31 |
| **Peptide 9.7.8** | **PQLAVSVILRVAKGYQE** | **SEQ ID NO: 2** |
| Peptide 9.7.9 | QLAVSVILRVAKGYQEL | SEQ ID NO: 32 |
| Peptide 9.7.10 | LAVSVILRVAKGYQELL | SEQ ID NO: 33 |
| Peptide 9.7.11 | LAVSVILRVAKGYQELLE | SEQ ID NO: 34 |
| Peptide 9.7.12 | PQLAVSVILRVAKGYQEL | SEQ ID NO: 35 |
| Peptide 9.7.13 | QLAVSVILRVAKGYQELL | SEQ ID NO: 36 |
| Peptide 9.7.14 | QLAVSVILRVAKGYQELLE | SEQ ID NO: 37 |
| Peptide 9.7.15 | PQLAVSVILRVAKGYQELL | SEQ ID NO: 38 |

Then, the same immunoassay was performed with the same samples as described above in **Example 2.**

### Example 7. Third assay results.

The optic density obtained in the ELISA are summarized in **Table 6** below. The mean of optical densities obtained by the different peptides in the group of patients was analyzed. This mean was higher for peptide 9.7.8 (0,217), which indicates that a greater number of antibodies bind to this sequence compared to the rest of the analyzed sequences. Therefore, peptide that shows the best results is peptide 9.7.8 (SEQ ID NO: 2).

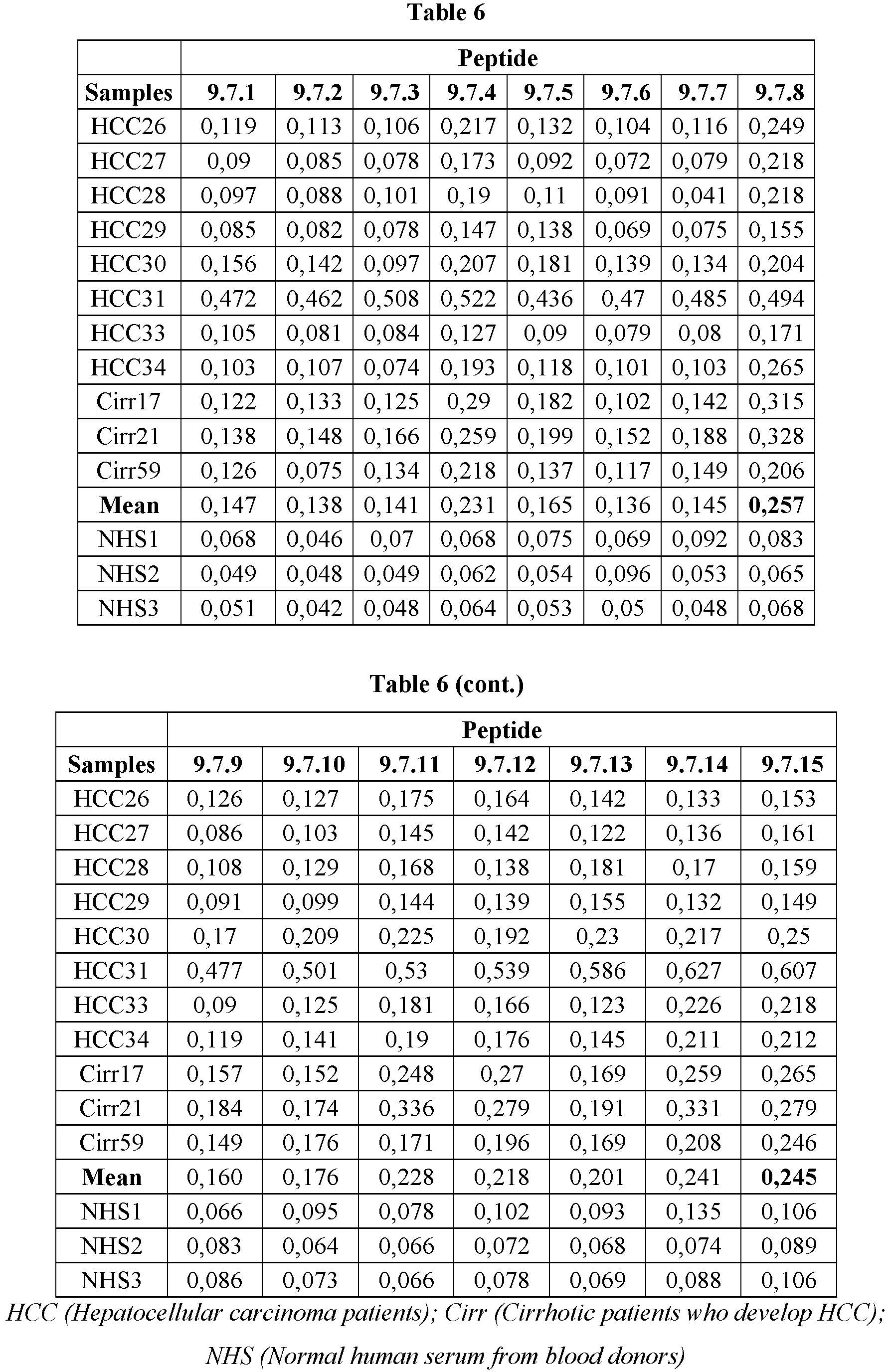

With the present results, we can conclude that the immunodominant epitope recognized by the samples of patients with HCC corresponds to the sequence of the peptide 9.7 of SEQ ID NO: 3, which include the SEQ ID NO: 2 and in turn the SEQ ID NO: 1.

### Example 8. Comparison between recombinant human AFP and AFP identified epitope.

Subsequently, we performed the immunoassay with the 9.7 peptide with the methodology described above and a capture immunoassay with the recombinant human AFP in patients with HCC as a pathologic population and active chronic hepatitis without cirrhosis as a control population.
To perform the capture immunoassay for recombinant human AFP we followed the protocol below:
ELISA Plate sensitization with anti-AFP mouse monoclonal antibody:
   - Non-covalent binding ELISA plate for hydrophilic amino acids Immulon 4 HBX (Thermofisher Scientific) for sandwich ELISA
   - 1/100 dilution of anti-AFP mouse monoclonal antibodies (mAb) (Bio-Rad) to have a concentration of 20 µg/mL, in 50 mM carbonate-bicarbonate buffer pH 9 were prepared.
   - Addition of 100 µL of anti-AFP mouse mAb dilution were incubated overnight at 4°C (3 µg/well)
   - After that, discard mouse mAb dilution and blocked the ELISA plate with 5% of bovine serum albumin in PBS
   - Perform 3 washes with PBS-Tween 20 0.05%
Antigen addition:
   - 100 µL of rhAFP at 10 µg/mL in PBS were added per well (1 µg/well)
   - Incubation of AFP for 1 hour at room temperature
   - Perform 3 washes with PBS
Perform ELISA assay:
   - Patients and control sera were diluted 1/100 with PBS-Tween 20 0.05%
   - 100 µL of diluted sera were added for rAFP treated and not treated per well and incubated for 1 hour at room temperature
   - Perform 3 washes with PBS-Tween 20 0.05%
   - 100 µL of anti-human IgG for cardiolipins (Inova Diagnostics) were incubated for 1 hour at room temperature
   - Perform 3 washes with PBS-Tween 20 0.05%
   - 100 µL of substrate (Inova Diagnostics) were incubated for 30 minutes at room temperature
   - Addition of 100 µL of ELISA STOP solution (Inova Diagnostics)
   - Spectrophotometer reading at 450 nm and reference filter at 620 nm

The optic density obtained in the ELISA are summarized in **Table 7** below:

**Table 7**

| **Samples** | **Peptide 9.7** | **rhAFP** |
|---|---|---|
| HCC1 | 1,180 | 0,577 |
| HCC2 | 1,570 | 0,272 |
| HCC3 | 0,680 | 0,316 |
| HCC4 | 1,690 | 0,235 |
| HCC5 | 1,070 | 0,275 |
| HCC6 | 1,130 | 0,269 |
| HCC7 | 1,060 | 0,223 |
| HCC8 | 0,500 | 0,169 |
| HCC9 | 2,010 | 0,126 |
| HCC10 | 1,120 | 0,256 |
| HCC11 | 1,500 | 0,554 |
| HCC12 | 0,800 | 0,272 |
| HCC13 | 0,580 | 0,329 |
| HCC14 | 0,460 | 0,188 |
| HCC15 | 0,830 | 0,552 |
| HCC16 | 0,510 | 0,468 |
| HCC17 | 1,320 | 0,296 |
| HCC18 | 0,570 | 0,135 |
| HCC19 | 0,770 | 0,175 |
| HCC20 | 0,680 | 0,473 |
| HCC21 | 0,650 | 0,255 |
| HCC22 | 0,540 | 0,626 |
| HCC23 | 0,660 | 0,504 |
| HCC24 | 0,910 | 0,541 |
| HCC25 | 1,110 | 0,209 |
| HCC26 | 0,790 | 0,367 |
| HCC27 | 1,060 | 0,265 |
| HCC28 | 1,210 | 0,430 |
| HCC29 | 0,410 | 0,227 |
| HCC30 | 0,710 | 0,684 |
| HCC31 | 1,140 | 0,213 |
| HCC32 | 0,580 | 0,372 |
| HCC33 | 0,710 | 0,435 |
| HCC34 | 0,540 | 0,244 |
| HCC35 | 1,960 | 0,180 |
| HCC36 | 0,540 | 0,151 |
| HCC37 | 0,900 | 0,150 |
| HCC38 | 0,860 | 0,169 |
| HCC39 | 0,550 | 0,467 |
| HCC40 | 0,640 | 0,194 |
| HCC41 | 0,360 | 0,277 |
| HCC42 | 0,430 | 0,224 |
| HCC43 | 0,300 | 0,160 |
| HCC44 | 0,670 | 0,166 |
| HCC45 | 0,500 | 0,130 |
| HCC46 | 0,220 | 0,223 |
| HCC47 | 0,640 | 0,162 |
| HCC48 | 0,610 | 0,206 |
| HCC49 | 0,900 | 0,229 |
| HCC50 | 0,500 | 0,171 |
| HCC51 | 0,880 | 0,238 |
| HCC52 | 0,200 | 0,106 |
| HCC53 | 0,460 | 0,156 |
| HCC54 | 0,700 | 0,241 |
| HCC55 | 0,860 | 0,332 |
| HCC56 | 0,330 | 0,178 |
| HCC57 | 0,470 | 0,178 |
| HCC58 | 0,300 | 0,178 |
| HCC59 | 0,430 | 0,148 |
| CH1 | 0,160 | 0,102 |
| CH2 | 0,120 | 0,100 |
| CH3 | 0,160 | 0,088 |
| CH4 | 1,450 | 0,265 |
| CH5 | 0,150 | 0,152 |
| CH6 | 0,200 | 0,095 |
| CH7 | 0,130 | 0,105 |
| CH8 | 0,080 | 0,073 |
| CH9 | 0,180 | 0,090 |
| CH10 | 0,140 | 0,091 |
| CH11 | 0,180 | 0,110 |
| CH12 | 0,160 | 0,216 |
| CH13 | 0,080 | 0,193 |
| CH14 | 0,120 | 0,089 |
| CH15 | 0,080 | 0,049 |
| CH16 | 0,060 | 0,088 |
| CH17 | 0,240 | 0,060 |
| CH18 | 0,350 | 0,134 |
| CH19 | 0,180 | 0,080 |
| CH20 | 0,150 | 0,168 |
| CH21 | 0,150 | 0,083 |
| CH22 | 0,140 | 0,073 |
| CH23 | 0,080 | 0,081 |
| CH24 | 0,090 | 0,072 |
| CH25 | 0,110 | 0,118 |
| CH26 | 0,270 | 0,070 |
| CH27 | 0,110 | 0,055 |
| CH28 | 0,100 | 0,081 |
| CH29 | 0,150 | 0,112 |
| CH30 | 0,170 | 0,133 |
| CH31 | 0,070 | 0,110 |
| CH32 | 0,100 | 0,187 |
| CH33 | 0,260 | 0,164 |
| CH34 | 0,270 | 0,105 |
| CH35 | 0,110 | 0,114 |
| CH36 | 0,110 | 0,127 |
| CH37 | 0,080 | 0,152 |
| CH38 | 0,210 | 0,198 |
| CH39 | 0,080 | 0,107 |
| CH40 | 0,110 | 0,074 |
| CH41 | 0,100 | 0,065 |
| CH42 | 0,090 | 0,201 |
| CH43 | 0,110 | 0,134 |
| CH44 | 0,070 | 0,066 |
| CH45 | 0,110 | 0,107 |
| CH46 | 0,150 | 0,130 |
| CH47 | 0,060 | 0,070 |
| CH48 | 0,230 | 0,131 |
| CH49 | Not determined | 0,096 |

| | | |
|---|---|---|
| *HCC (Hepatocellular carcinoma patients); CH (Chronic hepatitis patients without cirrhosis)* | | |

A ROC curve was performed with the optic density obtained from HCC patients and controls to establish the cut-off point to perform the analysis of the sensitivity, specificity and accuracy of both assays shown in **Table 8**:

**Table 8**

| | Cutoff point (Optic density) | Sensitivity | Specificity | Accuracy |
|---|---|---|---|---|
| HCC vs controls rhAFP | 0,134 | 94,9 | 79,6 | 0,931 |
| **HCC vs controls peptide 9.7** | 0,284 | **96,6** | **95,8** | **0,975** |

## Claims

1. Epitope consisting of SEQ ID NO: 1 or a sequence having an identity of at least 95% with the SEQ ID NO: 1.

2. Epitope, according to claim 1, consisting of SEQ ID NO: 2 or a sequence having an identity of at least 95% with the SEQ ID NO: 2.

3. Epitope, according to any of the claims 1 or 2, consisting of SEQ ID NO: 3 or a sequence having an identity of at least 95% with the SEQ ID NO: 3.

4. *In vitro* method for detecting and/or quantifying autoantibodies against alpha-fetoprotein (AFP) in a biological sample, wherein the autoantibodies bind to an epitope comprising the SEQ ID NO: 1, SEQ ID NO: 2 or SEQ ID NO: 3 which is immobilized on a solid support.

5. *In vitro* method for detecting and/or quantifying autoantibodies against alpha-fetoprotein (AFP) in a biological sample, according to claim 4, wherein the autoantibodies bind to an antigen comprising an epitope consisting of the SEQ ID NO: 1, SEQ ID NO: 2 or SEQ ID NO: 3 which is immobilized on a solid support.

6. *In vitro* method, according to any of the claims 3 to 5, wherein the detection and/or quantification of the autoantibodies is carried out by an immunochemical technique, preferably by ELISA, and most preferably by indirect ELISA.

7. *In vitro* method, according to any of the claims 3 to 6, which comprises:
a. Immobilizing an epitope comprising the SEQ ID NO: 1, SEQ ID NO: 2 or SEQ ID NO: 3 or an antigen comprising an epitope consisting of the SEQ ID NO: 1, SEQ ID NO: 2 or SEQ ID NO: 3 on a solid support,
b. Adding the biological sample to be analysed in the solid support,
c. Adding an antibody, conjugated with a detectable labelling agent, which reacts against the autoantibody which may be present in the biological sample,
d. Detecting and/or quantifying the complex obtained with a composition containing a chromogenic, fluorogenic and/or chemiluminescent indicator substrate.

8. *In vitro* method, according to any of the claims 3 to 7, wherein the sample is obtained from a subject who may be suffering from hepatocellular carcinoma.

9. *In vitro* method, according to any of claims 3 to 8, wherein the sample is blood, serum or plasma.

10. *In vitro* method for the diagnosis of hepatocellular carcinoma which comprises detecting and/or quantifying autoantibodies against alpha-fetoprotein (AFP) by following the method of any of the claims 3 to 9.

11. *In vitro* use of an epitope comprising the SEQ ID NO: 1, SEQ ID NO: 2 or SEQ ID NO: 3 or an antigen comprising an epitope consisting of the SEQ ID NO: 1, SEQ ID NO: 2 or SEQ ID NO: 3 for detecting and/or quantifying autoantibodies against alpha-fetoprotein (AFP).

12. *In vitro* use, according to claim 11, for the diagnosis of hepatocellular carcinoma.

13. Kit for detecting and/or quantifying autoantibodies against alpha-fetoprotein (AFP) in a biological sample which comprises:
a. An epitope comprising the SEQ ID NO: 1, SEQ ID NO: 2 or SEQ ID NO: 3 or an antigen comprising an epitope consisting of the SEQ ID NO: 1, SEQ ID NO: 2 or SEQ ID NO: 3.
b. At least an antibody which reacts against the autoantibody which may be present in the biological sample.

14. Kit, according to claim 13, for the diagnosis of hepatocellular carcinoma which comprises:
a. An epitope comprising the SEQ ID NO: 1, SEQ ID NO: 2 or SEQ ID NO: 3 or an antigen comprising an epitope consisting of the SEQ ID NO: 1, SEQ ID NO: 2 or SEQ ID NO: 3.
b. At least an antibody which reacts against the autoantibody which may be present in the biological sample.


SEQUENCE LISTING

<110>  CONSORCI CORPORACIÓ SANITÀRIA PARC TAULÍ

<120>  IMPROVED EPITOPE FOR DETECTING AND/OR QUANTIFYING
       ALPHA-FETOPROTEIN

<130>  905141

<160>  38

<170>  PatentIn version 3.5

<210>  1
<211>  14
<212>  PRT
<213>  Artificial sequence

<220>
<223>  Artificial sequence

<400>  1

Ala Val Ser Val Ile Leu Arg Val Ala Lys Gly Tyr Gln Glu
1               5                   10

<210>  2
<211>  17
<212>  PRT
<213>  Artificial sequence

<220>
<223>  Artificial sequence

<400>  2

Pro Gln Leu Ala Val Ser Val Ile Leu Arg Val Ala Lys Gly Tyr Gln
1               5                   10                  15

Glu

<210>  3
<211>  20
<212>  PRT
<213>  Artificial sequence

<220>
<223>  Artificial sequence

<400>  3

Pro Gln Leu Ala Val Ser Val Ile Leu Arg Val Ala Lys Gly Tyr Gln
1               5                   10                  15

Glu Leu Leu Glu
            20

<210>  4
<211>  48
<212>  PRT
<213>  Artificial sequence

<220>
<223>  Artificial sequence

<400>  4

Arg Thr Leu His Arg Asn Glu Tyr Gly Ile Ala Ser Ile Leu Asp Ser
1               5                   10                  15

Tyr Gln Cys Thr Ala Glu Ile Ser Leu Ala Asp Leu Ala Thr Ile Phe
            20                  25                  30

Phe Ala Gln Phe Val Gln Glu Ala Thr Tyr Lys Glu Val Ser Lys Met
        35                  40                  45

<210>  5
<211>  48
<212>  PRT
<213>  Artificial sequence

<220>
<223>  Artificial sequence

<400>  5

Thr Tyr Lys Glu Val Ser Lys Met Val Lys Asp Ala Leu Thr Ala Ile
1               5                   10                  15

Glu Lys Pro Thr Gly Asp Glu Gln Ser Ala Gly Cys Leu Glu Asn Gln
            20                  25                  30

Leu Pro Ala Phe Leu Glu Glu Leu Cys Arg Glu Lys Glu Ile Leu Glu
        35                  40                  45

<210>  6
<211>  48
<212>  PRT
<213>  Artificial sequence

<220>
<223>  Artificial sequence

<400>  6

Cys Arg Glu Lys Glu Ile Leu Glu Lys Tyr Gly His Ser Asp Cys Cys
1               5                   10                  15

Ser Gln Ser Glu Glu Gly Arg His Asn Cys Phe Leu Ala His Lys Lys
            20                  25                  30

Pro Thr Pro Ala Ser Ile Pro Phe Phe Gln Val Pro Glu Pro Val Thr

35                  40                  45

<210>  7
<211>  48
<212>  PRT
<213>  Artificial sequence

<220>
<223>  Artificial sequence

<400>  7

Phe Gln Val Pro Glu Pro Val Thr Ser Cys Glu Ala Tyr Glu Glu Asp
1               5                   10                  15

Arg Glu Thr Phe Met Asn Lys Phe Ile Tyr Glu Ile Ala Arg Arg His
            20                  25                  30

Pro Phe Leu Tyr Ala Pro Thr Ile Leu Leu Trp Ala Ala Arg Tyr Asp
        35                  40                  45

<210>  8
<211>  48
<212>  PRT
<213>  Artificial sequence

<220>
<223>  Artificial sequence

<400>  8

Leu Leu Trp Ala Ala Arg Tyr Asp Lys Ile Ile Pro Ser Cys Cys Lys
1               5                   10                  15

Ala Glu Asn Ala Val Glu Cys Phe Gln Thr Lys Ala Ala Thr Val Thr
            20                  25                  30

Lys Glu Leu Arg Glu Ser Ser Leu Leu Asn Gln His Ala Cys Ala Val
        35                  40                  45

<210>  9
<211>  48
<212>  PRT
<213>  Artificial sequence

<220>
<223>  Artificial sequence

<400>  9

Leu Asn Gln His Ala Cys Ala Val Met Lys Asn Phe Gly Thr Arg Thr
1               5                   10                  15

Phe Gln Ala Ile Thr Val Thr Lys Leu Ser Gln Lys Phe Thr Lys Val
            20                  25                  30

Asn Phe Thr Glu Ile Gln Lys Leu Val Leu Asp Val Ala His Val His
        35                  40                  45

<210>  10
<211>  48
<212>  PRT
<213>  Artificial sequence

<220>
<223>  Artificial sequence

<400>  10

Val Leu Asp Val Ala His Val His Glu His Cys Cys Arg Gly Asp Val
1               5                   10                  15

Leu Asp Cys Leu Gln Asp Gly Glu Lys Ile Met Ser Tyr Ile Cys Ser
            20                  25                  30

Gln Gln Asp Thr Leu Ser Asn Lys Ile Thr Glu Cys Cys Lys Leu Thr
        35                  40                  45

<210>  11
<211>  48
<212>  PRT
<213>  Artificial sequence

<220>
<223>  Artificial sequence

<400>  11

Ile Thr Glu Cys Cys Lys Leu Thr Thr Leu Glu Arg Gly Gln Cys Ile
1               5                   10                  15

Ile His Ala Glu Asn Asp Glu Lys Pro Glu Gly Leu Ser Pro Asn Leu
            20                  25                  30

Asn Arg Phe Leu Gly Asp Arg Asp Phe Asn Gln Phe Ser Ser Gly Glu
        35                  40                  45

<210>  12
<211>  48
<212>  PRT
<213>  Artificial sequence

<220>
<223>  Artificial sequence

<400>  12

Phe Asn Gln Phe Ser Ser Gly Glu Lys Asn Ile Phe Leu Ala Ser Phe
1               5                   10                  15

Val His Glu Tyr Ser Arg Arg His Pro Gln Leu Ala Val Ser Val Ile
            20                  25                  30

Leu Arg Val Ala Lys Gly Tyr Gln Glu Leu Leu Glu Lys Cys Phe Gln
        35                  40                  45

<210>  13
<211>  48
<212>  PRT
<213>  Artificial sequence

<220>
<223>  Artificial sequence

<400>  13

Glu Leu Leu Glu Lys Cys Phe Gln Thr Glu Asn Pro Leu Glu Cys Gln
1               5                   10                  15

Asp Lys Gly Glu Glu Glu Leu Gln Lys Tyr Ile Gln Glu Ser Gln Ala
            20                  25                  30

Leu Ala Lys Arg Ser Cys Gly Leu Phe Gln Lys Leu Gly Glu Tyr Tyr
        35                  40                  45

<210>  14
<211>  48
<212>  PRT
<213>  Artificial sequence

<220>
<223>  Artificial sequence

<400>  14

Phe Gln Lys Leu Gly Glu Tyr Tyr Leu Gln Asn Ala Phe Leu Val Ala
1               5                   10                  15

Tyr Thr Lys Lys Ala Pro Gln Leu Thr Ser Ser Glu Leu Met Ala Ile
            20                  25                  30

Thr Arg Lys Met Ala Ala Thr Ala Ala Thr Cys Cys Gln Leu Ser Glu
        35                  40                  45

<210>  15
<211>  48
<212>  PRT
<213>  Artificial sequence

<220>
<223>  Artificial sequence

<400>  15

Ala Thr Cys Cys Gln Leu Ser Glu Asp Lys Leu Leu Ala Cys Gly Glu
1               5                   10                  15

Gly Ala Ala Asp Ile Ile Ile Gly His Leu Cys Ile Arg His Glu Thr
            20                  25                  30

Thr Pro Val Asn Pro Gly Val Gly Gln Cys Cys Thr Ser Ser Tyr Ala
        35                  40                  45

<210>  16
<211>  48
<212>  PRT
<213>  Artificial sequence

<220>
<223>  Artificial sequence

<400>  16

Gln Cys Cys Thr Ser Ser Tyr Ala Asn Arg Arg Pro Cys Phe Ser Ser
1               5                   10                  15

Leu Val Val Asp Glu Thr Tyr Val Pro Pro Ala Phe Ser Asp Asp Lys
            20                  25                  30

Phe Ile Phe His Lys Asp Leu Cys Gln Ala Gln Gly Val Ala Leu Gln
        35                  40                  45

<210>  17
<211>  48
<212>  PRT
<213>  Artificial sequence

<220>
<223>  Artificial sequence

<400>  17

Gln Ala Gln Gly Val Ala Leu Gln Thr Met Lys Gln Glu Phe Leu Ile
1               5                   10                  15

Asn Leu Val Lys Gln Lys Pro Gln Ile Thr Glu Glu Gln Leu Glu Ala
            20                  25                  30

Val Ile Ala Asp Phe Ser Gly Leu Leu Glu Lys Cys Cys Gln Gly Gln
        35                  40                  45

<210>  18
<211>  33
<212>  PRT
<213>  Artificial sequence

<220>
<223>  Artificial sequence

<400>  18

Leu Glu Lys Cys Cys Gln Gly Gln Glu Gln Glu Val Cys Phe Ala Glu
1               5                   10                  15

Glu Gly Gln Lys Leu Ile Ser Lys Thr Arg Ala Ala Leu Gly Val Met
            20                  25                  30

Trp

<210>  19
<211>  20
<212>  PRT
<213>  Artificial sequence

<220>
<223>  Artificial sequence

<400>  19

Phe Asn Gln Phe Ser Ser Gly Glu Lys Asn Ile Phe Leu Ala Ser Phe
1               5                   10                  15

Val His Glu Tyr
            20

<210>  20
<211>  20
<212>  PRT
<213>  Artificial sequence

<220>
<223>  Artificial sequence

<400>  20

Ser Ser Gly Glu Lys Asn Ile Phe Leu Ala Ser Phe Val His Glu Tyr
1               5                   10                  15

Ser Arg Arg His
            20

<210>  21
<211>  20
<212>  PRT
<213>  Artificial sequence

<220>
<223>  Artificial sequence

<400>  21

Lys Asn Ile Phe Leu Ala Ser Phe Val His Glu Tyr Ser Arg Arg His
1               5                   10                  15

Pro Gln Leu Ala
            20

<210>  22
<211>  20
<212>  PRT
<213>  Artificial sequence

<220>
<223>  Artificial sequence

<400>  22

Leu Ala Ser Phe Val His Glu Tyr Ser Arg Arg His Pro Gln Leu Ala
1               5                   10                  15

Val Ser Val Ile
            20

<210>  23
<211>  20
<212>  PRT
<213>  Artificial sequence

<220>
<223>  Artificial sequence

<400>  23

Val His Glu Tyr Ser Arg Arg His Pro Gln Leu Ala Val Ser Val Ile
1               5                   10                  15

Leu Arg Val Ala
            20

<210>  24
<211>  20
<212>  PRT
<213>  Artificial sequence

<220>
<223>  Artificial sequence

<400>  24

Ser Arg Arg His Pro Gln Leu Ala Val Ser Val Ile Leu Arg Val Ala
1               5                   10                  15

Lys Gly Tyr Gln
            20

<210>  25
<211>  20
<212>  PRT

<213>  Artificial sequence

<220>
<223>  Artificial sequence

<400>  25

Val Ser Val Ile Leu Arg Val Ala Lys Gly Tyr Gln Glu Leu Leu Glu
1               5                   10                  15

Lys Cys Phe Gln
            20

<210>  26
<211>  15
<212>  PRT
<213>  Artificial sequence

<220>
<223>  Artificial sequence

<400>  26

Leu Ala Val Ser Val Ile Leu Arg Val Ala Lys Gly Tyr Gln Glu
1               5                   10                  15

<210>  27
<211>  15
<212>  PRT
<213>  Artificial sequence

<220>
<223>  Artificial sequence

<400>  27

Ala Val Ser Val Ile Leu Arg Val Ala Lys Gly Tyr Gln Glu Leu
1               5                   10                  15

<210>  28
<211>  16
<212>  PRT
<213>  Artificial sequence

<220>
<223>  Artificial sequence

<400>  28

Gln Leu Ala Val Ser Val Ile Leu Arg Val Ala Lys Gly Tyr Gln Glu
1               5                   10                  15

<210>  29
<211>  16
<212>  PRT
<213>  Artificial sequence

<220>

<223>  Artificial sequence

<400>  29

Leu Ala Val Ser Val Ile Leu Arg Val Ala Lys Gly Tyr Gln Glu Leu
1               5                   10                  15


<210>  30
<211>  16
<212>  PRT
<213>  Artificial sequence

<220>
<223>  Artificial sequence

<400>  30

Ala Val Ser Val Ile Leu Arg Val Ala Lys Gly Tyr Gln Glu Leu Leu
1               5                   10                  15


<210>  31
<211>  17
<212>  PRT
<213>  Artificial sequence

<220>
<223>  Artificial sequence

<400>  31

Ala Val Ser Val Ile Leu Arg Val Ala Lys Gly Tyr Gln Glu Leu Leu
1               5                   10                  15


Glu




<210>  32
<211>  17
<212>  PRT
<213>  Artificial sequence

<220>
<223>  Artificial sequence

<400>  32

Gln Leu Ala Val Ser Val Ile Leu Arg Val Ala Lys Gly Tyr Gln Glu
1               5                   10                  15


Leu




<210>  33
<211>  17
<212>  PRT
<213>  Artificial sequence

<220>
<223>  Artificial sequence

<400>  33

Leu Ala Val Ser Val Ile Leu Arg Val Ala Lys Gly Tyr Gln Glu Leu
1               5                   10                  15

Leu

<210>  34
<211>  18
<212>  PRT
<213>  Artificial sequence

<220>
<223>  Artificial sequence

<400>  34

Leu Ala Val Ser Val Ile Leu Arg Val Ala Lys Gly Tyr Gln Glu Leu
1               5                   10                  15

Leu Glu

<210>  35
<211>  18
<212>  PRT
<213>  Artificial sequence

<220>
<223>  Artificial sequence

<400>  35

Pro Gln Leu Ala Val Ser Val Ile Leu Arg Val Ala Lys Gly Tyr Gln
1               5                   10                  15

Glu Leu

<210>  36
<211>  18
<212>  PRT
<213>  Artificial sequence

<220>
<223>  Artificial sequence

<400>  36

Gln Leu Ala Val Ser Val Ile Leu Arg Val Ala Lys Gly Tyr Gln Glu
1               5                   10                  15

Leu Leu

<210> 37
<211> 19
<212> PRT
<213> Artificial sequence

<220>
<223> Artificial sequence

<400> 37

Gln Leu Ala Val Ser Val Ile Leu Arg Val Ala Lys Gly Tyr Gln Glu
1               5                   10                  15

Leu Leu Glu

<210> 38
<211> 19
<212> PRT
<213> Artificial sequence

<220>
<223> Artificial sequence

<400> 38

Pro Gln Leu Ala Val Ser Val Ile Leu Arg Val Ala Lys Gly Tyr Gln
1               5                   10                  15

Glu Leu Leu